# EUROPÄISCHE PATENTSCHRIFT

(11) **EP 0 839 211 B1**
(45) Veröffentlichungstag und Bekanntmachung des Hinweises auf die Patenterteilung: **06.06.2001**
(21) Anmeldenummer: 96926334.2
(22) Anmeldetag: 10.07.1996
(51) Int. Cl.: C12P 25/00, C12N 15/60, C12N 15/31

(54) **VERFAHREN ZUR HERSTELLUNG VON RIBOFLAVIN MITTELS MIKROORGANISMEN MIT VERÄNDERTER ISOCITRATLYASE-AKTIVITÄT**
RIBOFLAVIN-PRODUCTION PROCESS BY MEANS OF MICRO-ORGANISMS WITH MODIFIED ISOCITRATLYASE ACTIVITY
PROCEDE DE PRODUCTION DE RIBOFLAVINE AU MOYEN DE MICRO-ORGANISMES A ACTIVITE D'ISOCITRATLYASE MODIFIEE

(30) Priorität: 13.07.1995 DE 19525281; 06.12.1995 DE 19545468
(43) Veröffentlichungstag der Anmeldung: 06.05.1998
(73) Patentinhaber: BASF AKTIENGESELLSCHAFT, 67056 Ludwigshafen (DE); FORSCHUNGSZENTRUM JÜLICH GMBH, 52428 Jülich (DE)
(72) Erfinder: KÄSLER, Bruno, D-67071 Ludwigshafen (DE); SAHM, Hermann, D-52428 Jülich (DE); STAHMANN, Klaus-Peter, D-52428 Jülich (DE); SCHMIDT, Georg, D-52457 Aldenhoven (DE); BÖDDECKER, Theo, D-52428 Jülich (DE); SEULBERGER, Harald, D-69221 Dossenheim (DE)
(86) Internationale Anmeldenummer: EP9603009
(87) Internationale Veröffentlichungsnummer: WO9703208

(56) Entgegenhaltungen:
- EP-A- 0 405 370
- WO-A-93/03183
- MICROBIOLOGY, Bd. 142, Nr. 2, 1996, READING U.K., Seiten 411-417, XP002020315 SCHMIDT, G. ET AL.: "Inhibition of purified isocitrate lyase identified itaconate and oxalate as potentioal antimetabolites for the riboflavin overproducer Ashbya gossypii"
- MICROBIOLOGY, Bd. 142, Nr. 2, 1996, READING, U.K., Seiten 419-426, XP002020316 SCHNMIDT, G. ET AL.: "Correlation of isocitrate lyase activity and riboflavin formation in the riboflavin overproducer Ashbya gossypii"
- MOL. GEN. GENET., Bd. 241, 1993, Seiten 422-430, XP002020317 BARTH, G. UND SCHEUBER, T.: "Cloning of the isocitrate lyase gene (ICL1) from Yarrowia lipolytica and characterization of the deduced protein"

## Beschreibung

Die vorliegende Erfindung betrifft ein Verfahren zur Herstellung von Riboflavin mittels Mikroorganismen mit veränderter Isocitratlyase-Aktivität.

Das Vitamin B₂, auch Riboflavin genannt, ist für Mensch und Tier essentiell. Bei Vitamin-B₂-Mangel treten Entzündungen der Mundund Rachenschleimhäute, Risse in den Mundwinkeln, Juckreiz und Entzündungen in den Hautfalten u.ä. Hautschäden, Bindehautentzündungen, verminderte Sehschärfe und Trübung der Hornhaut auf. Bei Säuglingen und Kindern können Wachstumsstillstand und Gewichtsabnahme eintreten. Das Vitamin B₂ hat daher wirtschaftliche Bedeutung insbesondere als Vitaminpräparat bei Vitaminmangel sowie als Futtermittelzusatz. Daneben wird es auch als Lebensmittelfarbstoff, beispielsweise in Mayonnaise, Eiscreme, Pudding etc., eingesetzt.

Die Herstellung von Riboflavin erfolgt entweder chemisch oder mikrobiell. Bei den chemischen Herstellungsverfahren wird das Riboflavin in der Regel in mehrstufigen Prozessen als reines Endprodukt gewonnen, wobei relativ kostspielige Ausgangsprodukte - wie beispielsweise D-Ribose - eingesetzt werden müssen.

Eine Alternative zur chemischen Herstellung des Riboflavins bietet die Herstellung dieses Stoffes durch Mikroorganismen. Als Ausgangsprodukte für die mikrobielle Synthese können nachwachsende Rohstoffe, wie beispielsweise pflanzliche Öle, eingesetzt werden.

Die Herstellung von Riboflavin durch Fermentation von Pilzen wie Ashbya gossypii oder Eremothecium ashbyii ist bekannt (The Merck Index, Windholz et al., eds. Merck & Co., Seite 1183, **1983);** aber auch Hefen, wie z.B. Candida oder Saccharomyces, und Bakterien, wie Clostridium, sind zur Riboflavinproduktion geeignet. Riboflavin-überproduzierende Bakterienstamme sind beispielsweise in der EP **405370** beschrieben, wobei die Stämme durch Transformation der Riboflavin-Biosynthese-Gene aus Bacillus subtilis erhalten wurden. Diese Prokaryonten-Gene sind aber für ein rekombinantes Riboflavin-Herstellungsverfahren mit Eukaryonten wie Saccharomyces cerevisiae oder Ashbya gossypii ungeeignet.

In WO 93/03183 ist die Klonierung der für die Riboflavin-Biosynthese spezifischen Gene aus dem eukaryontischen Organismus Saccharomyces cerevisiae beschrieben. Mittels dieser Gene können rekombinante eukaryontische Mikroorganismen konstruiert werden, die eine effiziente Riboflavinproduktion gestatten.

Häufig liegen jedoch die Ausgangsprodukte und Substrate der Riboflavinbiosynthese-Enzyme in dem Mikroorganimus in limitierter Menge vor, so daß trotz Erhöhung der Riboflavinbiosynthese - Aktivität keine Steigerung in der Riboflavinproduktion erreicht wird.

Es bestand daher die Aufgabe ein verbessertes mikrobielles Verfahren zur Produktion von Riboflavin bereitzustellen, das Mikroorganismen verwendet, die keine oder eine geringere Substratlimitierung besitzen und somit eine erhöhte Riboflavinproduktion erlauben.

Die Aufgabe wird erfindungsgemäß dadurch gelöst, daß die verwendeten Mikroorganismen eine Veränderung in ihrer endogenen Isocitratlyaseaktivität besitzen.Die Veränderung ist gegenüber dem unveränderten Ausgangsstamm zu ermitteln. Es gibt eine Vielzahl von Möglichkeiten, solche Mikroorganismen mit veränderter ICL-Aktivität zu erhalten.

Eine Möglichkeit besteht darin, das endogene ICL-Gen so zu verändern, daß es für ein Enzym mit gegenüber dem Ausgangsenzym erhöhter ICL-Aktivität codiert. Eine Erhohung der Enzymaktivität kann beispielsweise erreicht werden, indem durch Veränderung des katalytischen Zentrums ein erhöhter Substratumsatz erfolgt oder indem die Wirkung von Enzyminhibitoren aufgehoben wird. Auch kann eine erhöhte Enzymaktivität durch Erhöhung der Enzymsynthese, beispielsweise durch Genamplifikation oder durch Ausschaltung von Faktoren, die die Enzymbiosynthese reprimieren, hervorgerufen werden. Die endogene ICL-Aktivität wird vorzugsweise durch Mutation des endogenen ICL-Gens erhöht. Derartige Mutationen können entweder nach klassischen Methoden ungerichtet erzeugt werden, wie beispielsweise durch UV-Bestrahlung oder mutationsauslösenden Chemikalien oder gezielt mittels gentechnischer Methoden wie Deletionen, Insertionen oder Substitutionen.

Die ICL-Genexpression wird durch Erhöhen der ICL-Genkopienzahl und / oder durch Verstärkung regulatorischer Faktoren , die die ICL-Genexpression positiv beeinflussen, erhöht. So kann eine Verstärkung regulatorischer Elemente vorzugsweise auf der Transkriptionsebene erfolgen, indem starke Transkriptionssignale wie Promotoren und Enhancer verwendet werden. Daneben ist aber auch eine Verstärkung der Translation möglich, indem beispielsweise die Stabilität der m-RNA verbessert wird. Zur Erhöhung der Genkopienzahl wird das ICL-Gen in ein Genkonstrukt bzw. in einen Vektor eingebaut, der vorzugsweise das dem ICL-Gen zugeordnete regulatorische Gensequenzen enthält, insbesondere solche, die die Genexpression verstärken. Anschließend wird ein Riboflavin-produzierenden Mikroorganismus mit dem das ICL-Gen enthaltende Genkonstrukt transformiert.

Das ICL-Gen wird vorzugsweise aus Mikroorganismen, insbesondere aus dem Pilz Ashbya gossypii, isoliert. Für die Isolierung des Gens kommen aber auch alle weiteren Organismen, deren Zellen die anaplerotische Sequenz des Glyoxylat-Cyclus und damit die Isocitratlyase enthalten, also auch Pflanzen, in Betracht. Die Isolierung des Gens kann durch homologe oder heterologe Komplementation einer im ICL-Gen defekten Mutante oder auch durch heterologes Probing oder PCR mit heterologen Primern erfolgen. Zur Subklonierung kann das Insert des komplementierenden Plasmids anschließend durch geeignete Schnitte mit Restriktionsenzymen in der Größe minimiert werden. Nach Sequenzierung und Identifizierung des putativen Gens erfolgt eine paßgenaue Subklonierung durch Fusions-PCR. Plasmide, die die so erhaltenen Fragmente als Insert tragen, werden in die ICL-Gen-defekte Mutante eingebracht, die auf Funktionalität des ICL-Gens getestet wird. Funktionelle Konstrukte werden schließlich zur Transformation eines Riboflavin-Produzenten eingesetzt.

Nach Isolierung und Sequenzierung sind Isocitratlyasegene mit Nukleotidsequenzen erhältlich, die für die in SEQ ID NO:2 angegebene Aminosäuresequenz oder deren Allelvariationen kodieren. Allelvariationen umfassen insbesondere funktionelle Derivate, die durch Deletion, Insertion oder Substitution von Nukleotiden aus der in SEQ ID NO:1 dargestellten Sequenz erhältlich sind, wobei die ICL-Aktivität aber erhalten bleibt.

Den Isocitratlyasegenen ist insbesondere ein Promotor der Nukleotidsequenz von Nukleotid 176 bis 550 gemäß SEQ ID NO:1 oder eine im wesentlichen gleichwirkende DNA-Sequenz vorgeschaltet. So kann beispielsweise dem Gen ein Promotor vorgeschaltet sein, der sich von dem Promotor mit der angegebenen Nukleotidsequenz durch ein oder mehrere Nukleotidaustausche, durch Insertion(en) und/ oder Deletion(en) unterscheidet, ohne daß aber die Funktionalität bzw. Wirksamkeit des Promotors beeinträchtigt ist. Des weiteren kann der Promotor auch durch Veränderung seiner Sequenz in seiner Wirksamkeit erhöht oder komplett durch wirksamere Promotoren ausgetauscht werden.

Dem ICL-Gen können des weiteren regulatorische Gensequenzen bzw. Regulatorgene zugeordnet sein, die insbesondere die ICL-Gen-Aktivität erhöhen. So können dem ICL-Gen beispielsweise sog. "enhancer" zugeordnet sein, die über eine verbesserte Wechselwirkung zwischen RNA-Polymerase und DNA eine erhöhte ICL-Genexpression bewirken.

Dem Isocitratlyasegen mit oder ohne vorgeschaltetem Promotor bzw. mit oder ohne Regulatorgen können ein oder mehrere DNA-Sequenzen vor- und/oder nachgeschaltet sein, so daß das Gen in einer Genstruktur enthalten ist.

Durch Klonierung des ICL-Gens sind Plasmide bzw. Vektoren erhältlich, die das ICL-Gen enthalten und - wie bereits oben erwähnt - zur Transformation eines Riboflavin-Produzenten geeignet sind. Die durch Transformation erhältlichen Zellen, bei denen es sich vorzugsweise um transformierte Zellen von Ashbya gossypii handelt, enthalten das Gen in replizierbarer Form, d.h. in zusätzlichen Kopien auf dem Chromosom, wobei die Genkopien durch homologe Rekombination an beliebigen Stellen des Genoms integriert werden, und/oder auf einem Plasmid bzw. Vektor.

Eine weitere Möglichkeit, Mikroorganismen mit veränderter ICL-Aktivität zu erzeugen, besteht darin, Mikroorganismen mit einer Resistenz gegennüber auf ICL hemmend wirkenden Substanzen zu erzeugen und diese zu selektionieren. Hemmstoffe der Isocitratlyase (ICL) sind dem Fachmann bekannt und beispielsweise in Handbook of Enzyme Inhibitors, Herausgeber: Hellmut Zollner, Verlag Chemie, Weinheim, 1993, auf Seite 291 aufgeführt. Besonders geeignete Hemmstoffe sind Phosphoenolpyruvat (PEP), 6-P-Gluconat, Maleat, insbesondere aber Itaconat und Oxalat.

Werden nunmehr Riboflavin produzierende Mikroorganismen-Stämme in Gegenwart solcher Hemmstoffe kultiviert, zeigt sich überraschenderweise, daß die Riboflavinbildung gehemmt ist. Dies äußert sich auf Kulturplatten in der Ausbildung von Kolonien, die nicht gelb werden, sondern weiß bleiben. Mit diesem System sind daher Stämme leicht erkennbar, die gegen eine Isocitratlyase-Hemmung resistent sind, da solche Stämme auch in Gegenwart von Hemmstoff Riboflavin bilden und daher gelb gefärbte Kolonien ausbilden. Derartige Stämme können entweder durch Spontanmutation entstehen oder indem entsprechende Mutationen durch gängige Methoden, wie beispielsweise chemisch oder durch UV-Bestrahlung, induziert werden. Es können somit Mikroorganismen-Stämme gewonnen werden, die einen erhöhten Anteil an Riboflavin in das Kulturmedium ausscheiden. Als resistenter Stamm mit erhöhter Riboflavinbildung wurde insbesondere der bei der DSM unter der Nr. 10067 hinterlegte Ashbya gossypii-Stamm erhalten.

Als Mikroorganismus werden in dem erfindungsgemäßen Verfahren bevorzugt Pilze eingesetzt. Geeignete Pilze sind beispielsweise solche, die in Indian Chem Engr. Section B. Vol 37, No 1,2 (1995) auf Seite 15, Tabelle 6 aufgeführt sind.

Insbesonders sind solche der Gattungen Pichia, Eremothetium und Ashbya, besonders Ashbya gossypii geeignet.

Es können aber auch andere Mikroorganismen als Pilze, beispielsweise Bakterien, insbesondere die, die in Indian Chem Engr. Section B. Vol 37, No 1,2 (1995) auf Seite 16, Tabelle 6 aufgeführt sind, eingesetzt werden.

### Beispiel 1:

### Erstellung einer genomischen Genbank aus Ashbya gossypii

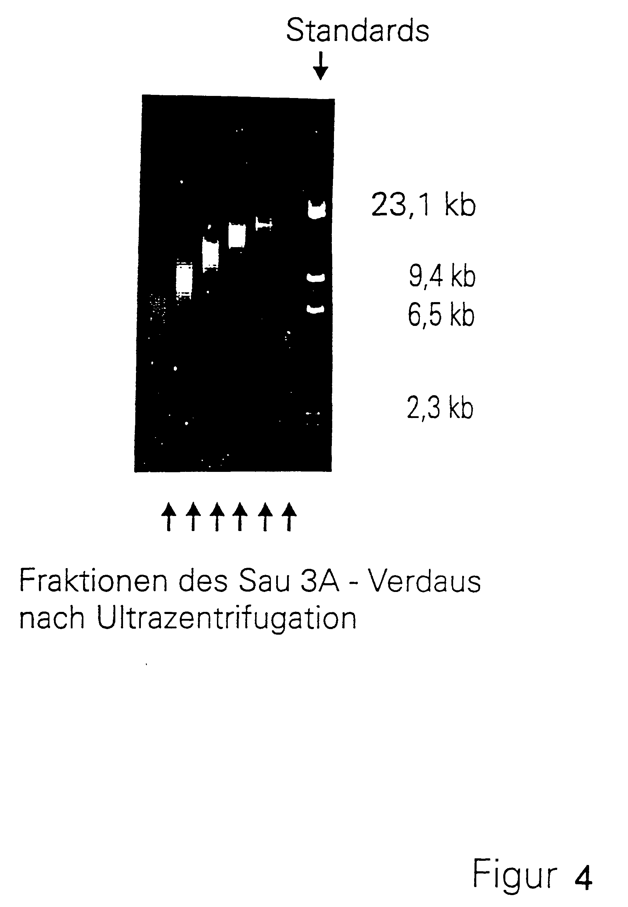

Zur Erstellung einer genomischen DNA-Bank wurde chromosomale DNA nach der Methode von Wright und Philippsen (1991, Gene 109: 99-105) isoliert. Die DNA wurde partiell mit Sau 3A verdaut und mit einem Saccharose-Dichtegradienten fraktioniert. Die größten Fragmente (Figur 4) wurden mit dem Bam HI geschnittenen E.coli,S.cerevisiae Shuttlevektor YEp 352 (J.E. Hill et al.,1993, Yeast 2: 163-167) ligiert. Mit diesem Ligationsansatz wurde E.coli DH5 a transformiert Von Platten mit Ampicillin und X-Gal wurden 3600 Kolonien isoliert, die durch ihre weiße Farbe als Klone mit Insert tragendem Plasmid erkennbar waren. Die Untersuchung von dreißig solcher zufällig ausgewählter Klone ergab, daß tatsächlich alle ein Plasmid trugen, diese Inserts im Größenbereich 7-18 kb hattten und alle Inserts verschieden waren, was anhand der Restiktionsmuster erkennbar war. Aufgrund einer Genomgröße von 7 x 10³ kb für Ashbya gossyii liegt die Wahrscheinlichkeit, das jedes Gen in dieser Genbank enthalten ist, bei 97 % - 99,99 %. Je 100 Klone wurden auf einer Agarplatte in großen Ausstrichen kultiviert und danach die Plasmide als Pool präpariert. Die Genbank bestand dementsprechend aus 36 Plasmidpools.

### Beispiel 2:

### Selektion des icl1-tragenden Genbankfragments

Mit den Plasmidpräparationen der Genbank wurde die Hefe Saccharomyces cerevisiae ICL1d ura3(fs) (E. Fernández et al., 1992, Eur. J. Biochem. 204: 983-990) transformiert. Diese Mutante ist im ICL1-Gen disruptiert und besitzt im ura3-Gen eine Mutation im Leserahmen. Dieser Genotyp fuhrt dazu, daß der Stamm nicht auf Ethanol als Kohlenstoffquelle wachsen kann und eine Uracil-Auxotrophie zeigt. Im ersten Schritt wurden die mit der Genbank transformierten Hefezellen auf Minimalmedium mit Glucose als einziger Kohlenstoffquelle selektioniert. Aufgrund des auf dem Plasmid vorhandenen ura3-Gens konnten nur die Zellen wachsen, die ein Plasmid aufgenommen hatten, denn das Minimalmedium enthielt kein Uracil. In diesem Schritt wurden 1900 Klone erhalten. Diese wurden durch Replikaplattierung auf ein Minimalmedium mit Ethanol als einziger Kohlenstoffquelle übertragen. Da zum Wachstum auf Ethanol unbedingt die Isocitratlyase als anaplerotisches Enzym nötig ist, konnten nur die Klone wachsen, die auf dem Plasmid das ICL-Gen trugen. Es konnten zwei Klone isoliert werden, die auf Ethanol wuchsen.

### Beispiel 3:

### Überprüfung der Funktionalität des isolierten Genbankfragments

Zur Überprüfung, ob die Komplementierung des chromosomalen ICL-Defekts plasmid-kodiert war, wurden die selektionierten Saccharomyces-Klone zweimal auf Vollmedium mit Uracil kultiviert und die erhaltenen Zellen auf Platten vereinzelt. Von 16 bzw. 13 zufällig ausgewählten Klonen wuchsen 7 bzw. 5 nicht mehr auf Minimalmedium mit Glucose. Genau diese Klone wuchsen auch nicht mehr auf Minimalmedium mit Ethanol. Die Kurierung vom Plasmid war also mit dem Verlust der ICL1d-Komplementation korreliert.

Aus einem der beiden Klone wurde das Plasmid wieder isoliert Es enthielt ein Insert von etwa 8 kb. Erneute Transformation der Saccharomyces. Mutante führte zur Komplementation aller gefundenen Klone. Das 8 kb - Fragment ließ sich durch Sph I auf 2,9 kb, die voll funktionell waren, verkürzen.

Im Rohextrakt der auf Ethanol gewachsenen Transformande war die Isocitratlyase mit einer spezifischen Aktivität von 0,3 U/mg Protein meßbar. Zudem zeigte der Westernblott mit polyklonalen Antikörpern gegen die Ashbya-ICL ein deutliches Signal.

PCR mit von tryptischen Peptiden der ICL abgeleiteten Primern ergab starke Signale der erwarteten Größe. Aus einem zweidimensionalen Elektrophoresegel wurde ein Protein isoliert, mit Trypsin in Peptide zerlegt und durch Edmannabbau ansequenziert. Der Vergleich der Peptidsequenzen mit Datenbanken ergab eine Identität von über 70 % mit der Isocitratlyase aus Saccharomyces cerevisiae. Davon abgeleitete Primer wurden zur PCR eingesetzt. Von dem ca. 8 kb großen komplementierenden Genbankfragement wurden 3,3 kb sequenziert (Sanger et al. Proc. Natl. Acad. Sci. USA 74 (1977) 5463-5467). Auf der ermittelten Sequenz konnten durch Datenbankvergleich zwei kodierende Bereiche gefunden werden. Ein Leserahmen von 1680 Basen (SEQ ID NO:1) zeigt eine 65 %ige Identität zum ICL1-Gen von Saccharomyces cerevisiae. Das ICL-Gen liegt 375 Basen upstream von einer Sequenz die 84 % Identität zu einer Ser-tRNA von Saccharomyces cerevisiae zeigt (SEQ ID NO:1).

### Beispiel 4:

### Funktionalität subklonierter ICL in einem E.coli/Hefe/Ashbya - Shuttlevektor

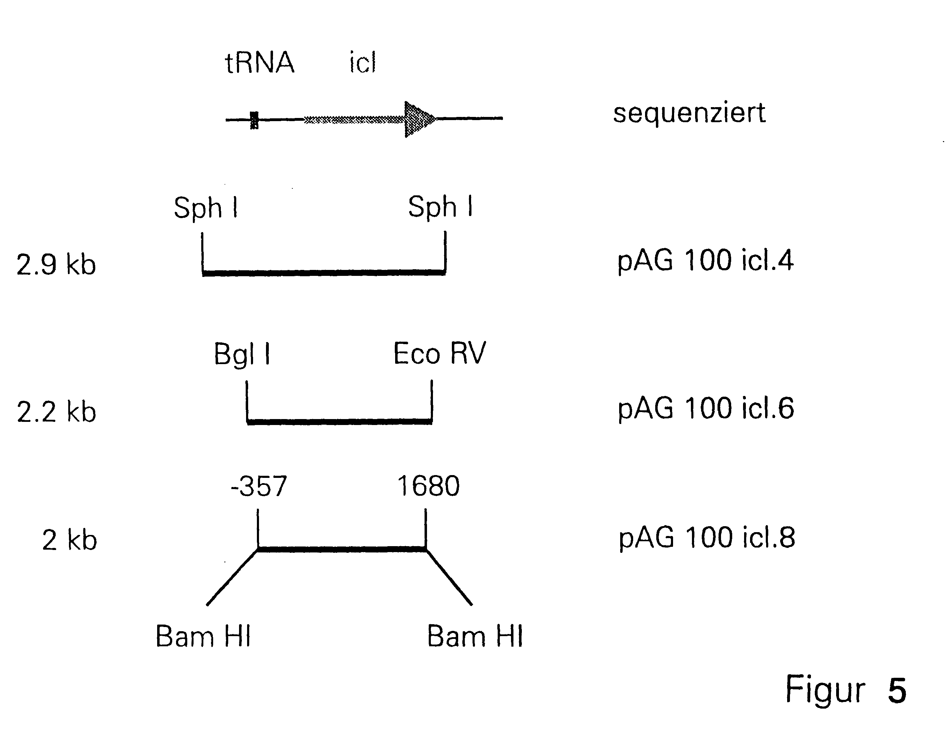

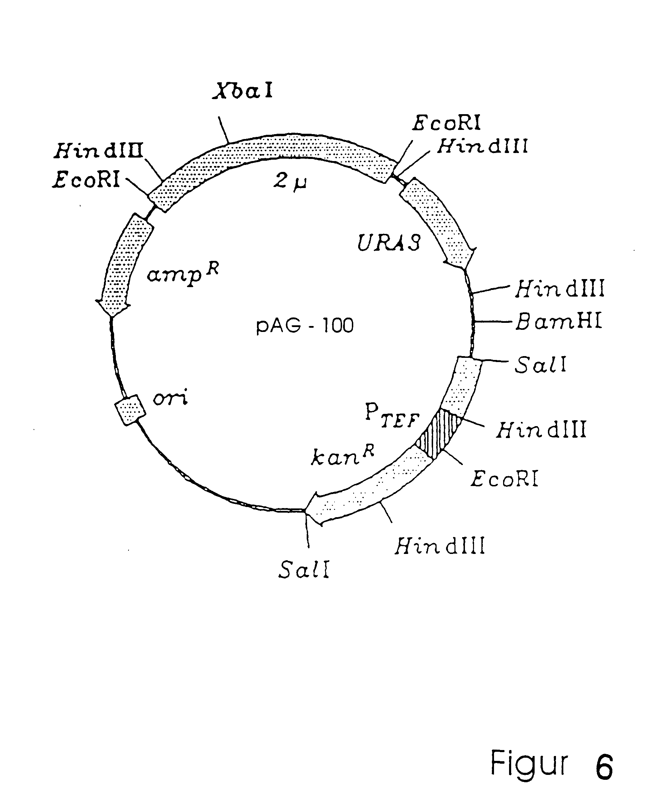

Zwei durch Restriktionsverdau erhaltene Fragmente und ein PCR-Produkt des isolierten Genbankfragments (Figur 5) wurden in das von Steiner und Philippsen (1994, Mol. Gen. Genet 242: 263-271) konstruierte Plasmid pAG 100 (Figur 6) kloniert. Bei den Fragmenten handelte es sich um ein 2.9 kb Sph I- Fragment ( pAG 100 icl.4) und um ein 2.2 kb Bgl 1 / Eco RV - Fragment (pAG 100 icl.6). Beide Fragment enthielten die Ser-tRNA. Deshalb wurde zusätzlich eine PCR-Amplifikation des putativen Gens mit daran fusionierten Bam HI - Schnittstellen (pAG 100 icl.8) durchgeführt. Alle drei DNAs wurden in die Bam HI site des Plasmids pAG 100 kloniert. Mit den erhaltenen Plasmiden wurde die Hefemutante Saccharomyces cerevisiae ICL1d ura3 (fs) transformiert. Alle drei Konstrukte führten zur vollständigen Komplementation der ICLld-Disruption d.h. trugen funktionelle Gene.

### Beispiel 5:

### Wirkung der ICL tragenden Plasmide auf die Riboflavinbildung von Ashbya gossypii

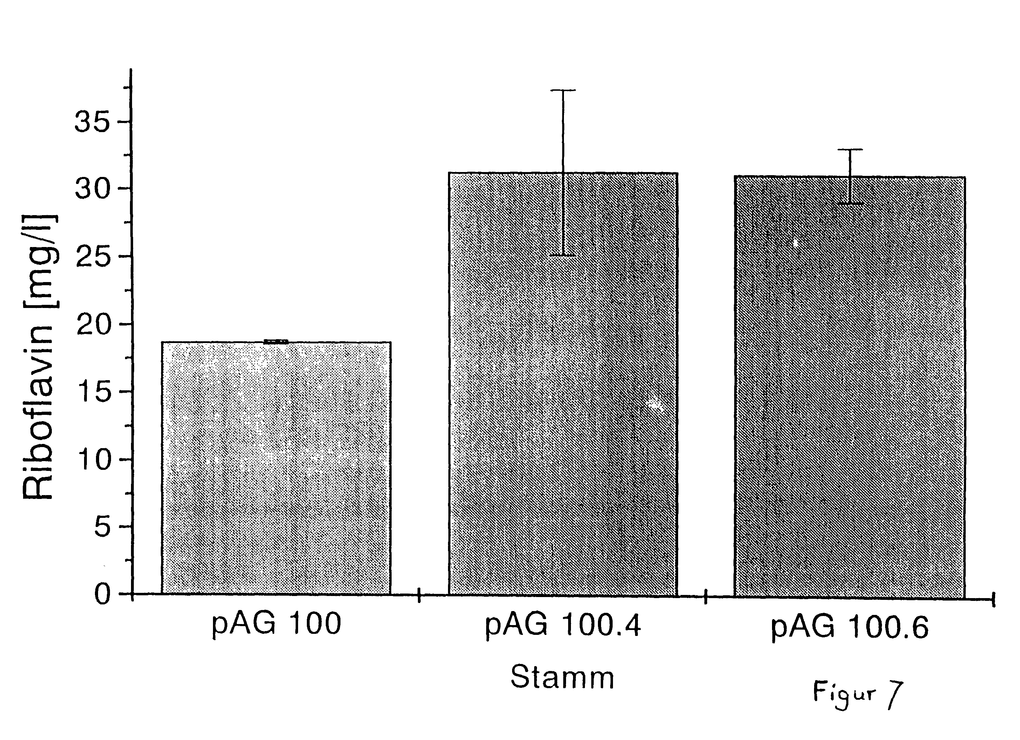

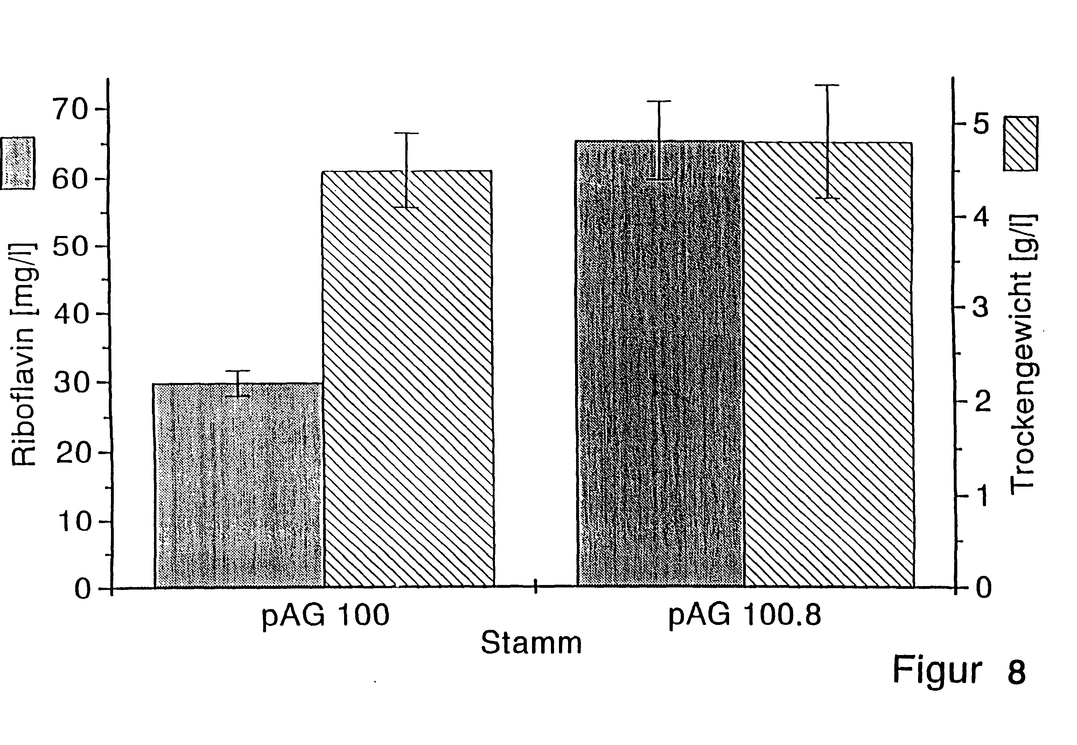

Die Transformation von Ashbya gossypii (Methode: Wright und Philippsen, 1991, Gene 109: 99-105) mit den oben erklärten Plasmiden führte zu signifikanten Erhöhungen der Riboflavinbildung. Kultiviert wurde in 500 ml Schüttelkolben mit zwei Schikanen, das 50 ml Medium aus 10 g/I Sojaöl, 10 g/I Hefeextrakt und 200 µg/ml Geneticin enthielt. Der Kontrollstamm A.gossypii pAG 100, der ein Plasmid ohne Insert enthielt, produzierte in zwei Tagen 18,7 ± 0,1 mg/l Riboflavin. Die Stämme A. gossypii pAG 100.4 und Agossypii pAG 100.6 produzierten 31,2 ± 6,1 mg/I bzw. 31,0 ± 2,0 mg(I Riboflavin (Figur 7). Eine signifikante Anderung der spezifischen Aktivität der Isocitratlyase war aufgrund der starken Streuung nicht messbar. Der Stamm A. gossypii pAG 100.8 produzierte in einem Medium, das noch durch 3 g/I Glycin supplementiert wurde, innerhalb von drei Tagen 65 ± 5,6 mg/I Riboflavin. Der Kontrollstamm A.gossypii pAG 100 bildete dagegen im direkten Vergleich nur 29,9 ± 1,8 mg/l Riboflavin (Figur 8). Weder in der spezifischen Aktivität der Isocitratlyase noch im Myzeltrockengewicht waren signifikante Unterschiede meßbar.

### Beispiel 6:

### Reinigung einer Isocitratlyase (ICL)

Zur Identifizierung von auf ICL hemmend wirkenden Substanzen wurde zunächst die ICL aus Ashbya gossypii gereinigt. Die Isolierung und Reinigung des Enzyms erfolgte 10nach Wachstum des Pilzmycels auf Pflanzenöl. Die einzelnen Reinigungsschritte sind in der Tabelle 1 zusammengefaßt: Demgemäß enthält ein typischer, aus ca. 25 g Mycel hergestellter Rohextrakt, der durch Zellaufschluß mit einer French-Press gewonnen wurde, 220 Einheiten ICL-Aktivität. Etwa 78% davon sind nach Zentrifugation bei 40.000 g gelöst im Überstand wiederzufinden. Eine anschließende fraktionierte Ammoniumsulfatfällung führt zu einer dreifachen Anreicherung des Enzyms. Nach einer Gelfiltration mit einer Sephacryl S-300 Säule wird die TCL an den Kationenaustauscher Mono S-Sepharose gebunden und mit NaCl eluiert. Das so erhaltene Präparat ist homogen in der SDS-Polyacrylamidgelelektrophorese und hat eine spezifische Aktivität von 18,4 U/mg.

### Beispiel 7:

### Identifizierung von ICL-Hemmstoffen

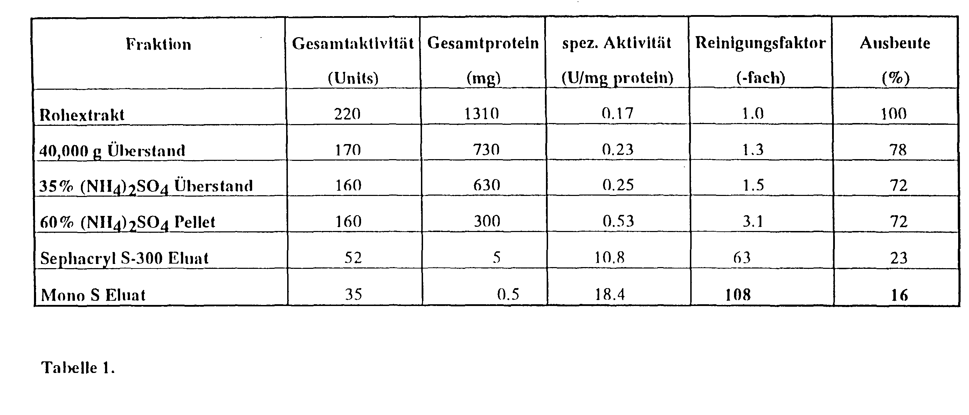

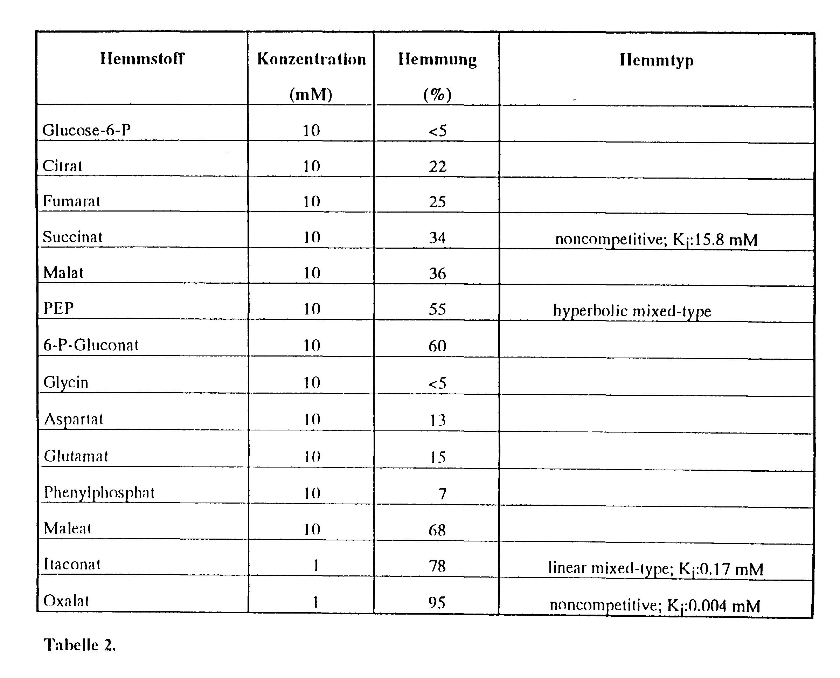

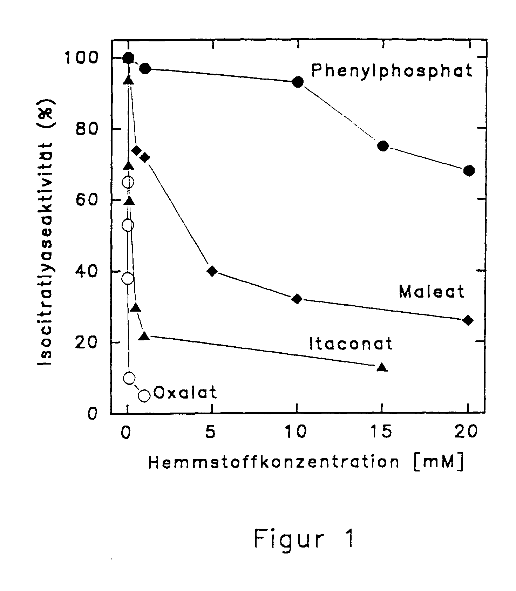

Mit dem gereinigten Enzym lassen sich in einem colorimetrischen Test (Dixon, H. und Kornberg, H.L. (1959), Biochem. J. 72, 3: Assay methods for key enzymes of the glyoxylate cycle) Einflüsse von Substanzen auf die Aktivität messen. In Tabelle 2 und Figur 1 sind die Effekte der getesteten Substanzen auf das Enzym zusammengefaßt bzw. dargestellt. Untersucht wurden zum einen Substanzen, die als Metaboliten in der Pilzzelle einen hemmenden Ef fekt auf das Enzym haben könnten. Darunter zeigten 6-P-Gluconat und Phosphoenolpyruvat die deutlichsten Hemmwirkungen mit über 50% bei einer Konzentration von 10 mM. Erheblich besser wirkten jedoch Itakonat und Oxalat, die vermutlich nicht im Stoffwechsel des Pilzes vorkommen. Bereits eine Konzentration von 1 mmol führte zu 78% bzw. 95% Hemmung.

### Beispiel 8:

### Charakterisierung einer mit Itakonat selektionierten Mutante

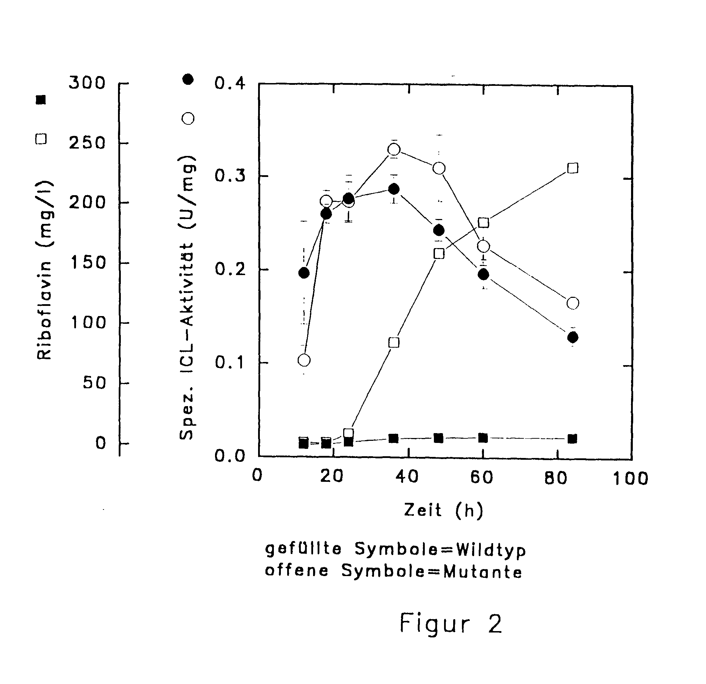

Durch UV-Bestrahlung von isolierten Sporen des Pilzes lassen sich Mutationen im Erbmaterial erzeugen. Mit einer Strahlendosis, bei der 10-20% der eingesetzten Sporen überleben, erhält man Mutanten, die gegen eine Hemmung der Riboflavinbildung durch Itakonat resistent sind. Eine so isolierte Mutante zeigt bei Wachstum auf Sojaöl eine 25-fache Riboflavinbildung im Vergleich zum Ausgangsstamm (Figur 2). Die spezifische ICL-Aktivität ist während der Ribof lavinbildungsphase um bis zu 15% erhöht (Figur 2). Mit Antikörpern läßt sich zeigen, daß die Proteinmenge erhöht ist. Die ICL aus der Mutante zeigt das gleiche Hemmverhalten durch Itakonat wie der Ausgangsstamm.

### Beispiel 9:

### Korrelation von Riboflavinbildung und spezifischer ICL-Aktivität

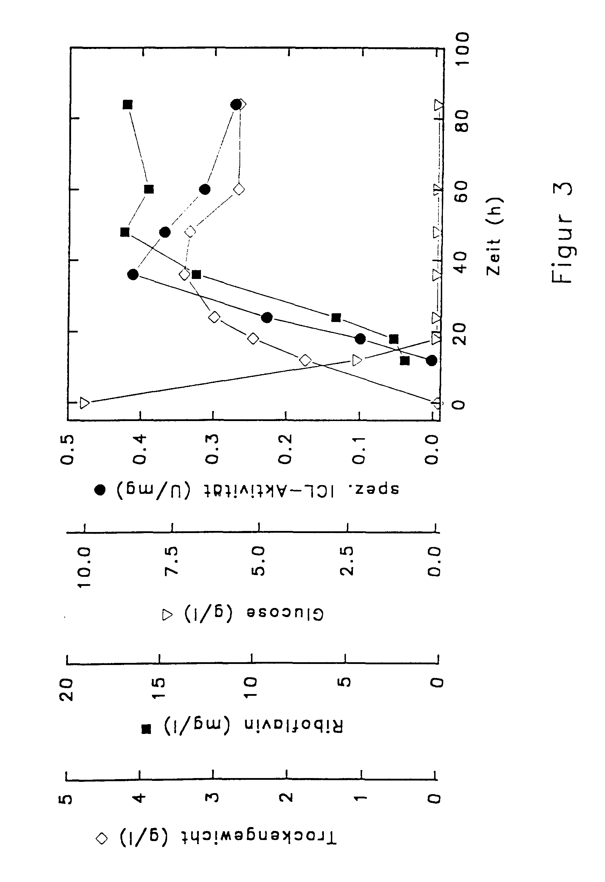

Einen überraschenden Hinweis auf einen kausalen Zusammenhang zwischen ICL und Riboflavinbildung liefert die Beobachtung, daß der Pilz, wenn Glucose als Substrat angeboten wird, erst nach Verbrauch der Glucose mit der Produktion beginnt. Genau dann wird auch die ICL, die zuvor durch Glucose reprimiert ist, im Rohextrakt meßbar und steigt bis zu Aktivitäten, wie sie bei Wachstum auf Öl gefunden werden, an (Figur 3).

## Patentansprüche

1. Verfahren zur mikrobiellen Herstellung von Riboflavin durch Kultivierung von Riboflavin produzierenden Mikroorganismen in einem Nährmedium und anschließender Isolierung des hergestellten Riboflavins, dadurch gekennzeichnet, daß Mikroorganismen verwendet werden, bei denen die endogene Isocitratlyase (ICL) Aktivität verändert wurde.

2. Verfahren nach Anspruch 1, dadurch gekennzeichnet, daß die Mikroorganismen durch Mutation des endogenen ICL-Gens ein Enzym mit höherer ICL-Aktivität aufweisen.

3. Verfahren nach Anspruch 1, dadurch gekennzeichnet, daß die Mikroorganismen durch eine Erhöhung der ICL-Genkopienzahl eine höhere ICL-Genexpression besitzen.

4. Verfahren nach Anspruch 1, dadurch gekennzeichnet, daß das ICL-Gen mit regulatorischen DNA-Sequenzen funktionell verknüpft wurde, die eine verstärkte Genexpression des ICL-Gens erlauben.

5. Verfahren nach Anspruch 1, dadurch gekennzeichnet, daß Mikroorganismen mit Resistenz gegenüber auf ICL hemmend wirkenden Substanzen verwendet werden.

6. Verfahren nach Anspruch 5, dadurch gekennzeichnet, daß die Mikroorganismen resistent gegenüber den Stoffen Itakonat oder Oxalat sind.

7. Verfahren nach einem der Ansprüche 1 bis 6, dadurch gekennzeichnet, daß als Mikroorganismus ein Pilz verwendet wird.

8. Verfahren nach Anspruch 7, dadurch gekennzeichnet, daß der Pilz aus der Gattung Ashbya verwendet wird.

9. ICL-Gen codierend für die in SEQ ID NO: 2 dargestellte Aminosäureseauenz.

10. Genkonstrukt enthaltend ein ICL-Gen gemäß Anspruch 9.

11. Genkonstrukt nach Anspruch 10, dadurch gekennzeichnet, daß das ICL-Gen funktionell mit einem oder mehreren Regulationssignalen zur Erhöhung der Genexpression funktionell verknüpft wurde.

## Claims

1. A process for the microbial preparation of riboflavin by culturing riboflavin-producing microorganisms in a nutrient medium and then isolating the riboflavin which has been produced, which comprises using microorganisms in which the endogenous isocitrate lyase (ICL) activity has been altered.

2. A process as claimed in claim 1, wherein the microorganisms exhibit an enzyme having a higher ICL activity due to mutation of the endogenous ICL gene.

3. A process as claimed in claim 1, wherein the microorganisms possess a higher ICL gene expression due to an increase in the copy number of the ICL gene.

4. A process as claimed in claim 1, wherein the ICL gene has been functionally linked to regulatory DNA sequences which enable expression of the ICL gene to be reinforced.

5. A process as claimed in claim 1, wherein use is made of microorganisms which exhibit resistance to substances which have an inhibitory effect on ICL.

6. A process as claimed in claim 5, wherein the microorganisms are resistant to the substances itaconate and oxalate.

7. A process as claimed in any one of claims 1 to 6, wherein a fungus is used as the microorganism.

8. A process as claimed in claim 7, wherein a fungus from the Ashbya genus is used.

9. An ICL gene encoding the amino acid sequence which is depicted in SEQ ID NO: 2.

10. A gene construct comprising an ICL gene as claimed in claim 9.

11. A gene construct as claimed in claim 10, wherein the ICL gene has been functionally linked to one or more regulatory signals for the purpose of increasing expression of the gene.

## Revendications

1. Procédé pour la préparation microbienne de riboflavine par culture de micro-organismes produisant de la riboflavine dans un milieu nutritif et ensuite isolement de la riboflavine produite, caractérisé par le fait qu'on utilise des micro-organismes dans lesquels l'activité de l'isocitratelyase endogène (ICL) est modifiée.

2. Procédé selon la revendication 1, caractérisé par le fait que les micro-organismes présentent une enzyme ayant une activité d'ICL plus élevée par mutation du gène d'ICL endogène.

3. Procédé selon la revendication 1, caractérisé par le fait que les micro-organismes possèdent une expression du gène d'ICL plus élevée par une augmentation du nombre des copies du gène d'ICL.

4. Procédé selon la revendication 1, caractérisé par le fait que le gène d'ICL a été couplé fonctionnellement avec des séquences d'ADN régulatrices, qui permettent une expression génique renforcée du gène d'ICL.

5. Procédé selon la revendication 1, caractérisé par le fait qu'on utilise des micro-organismes ayant une résistance vis-à-vis des substances ayant une action inhibitrice sur l'ICL.

6. Procédé selon la revendication 5, caractérisé par le fait que les micro-organismes sont résistants vis-à-vis des substances itaconate ou oxalate.

7. Procédé selon l'une des revendications 1 à 6, caractérisé par le fait qu'on utilise un champignon comme micro-organisme.

8. Procédé selon la revendication 7, caractérisé par le fait qu'on utilise le champignon de l'espèce Ashbya.

9. Gène d'ICL codant pour la séquence d'acides aminés représentée par SEQ ID NO: 2.

10. Construit de gène contenant un gène d'ICL selon la revendication 9.

11. Construit de gène selon la revendication 10, caractérisé par le fait que le gène d'ICL a été couplé fonctionnellement avec un ou plusieurs signaux de régulation pour augmenter l'expression génique.


### SEQUENZPROTOKOLL

(1) ALGEMEINE INFORMATION:
   (i) ANMELDER:
      (A) NAME: BASF Aktiengesellschaft
      (B) STRASSE: Carl-Bosch-Strasse 38
      (C) ORT: Ludwigshafen
      (E) LAND: Bundesrepublik Deutschland
      (F) POSTLEITZAHL: D-67056
      (G) TELEPHON: 0621/6048526
      (H) TELEFAX: 0621/6043123
      (I) TELEX: 1762175170

      (A) NAME: Forschungszentrum Juelich GmbH
      (B) STRASSE: Leo-Brandt-Strasse
      (C) ORT: Juelich
      (E) LAND: Germany
      (F) POSTLEITZAHL: D-52425
      (G) TELEPHON: 02461-61 3004
   (ii) ANMELDETITEL: Verfahren zur Herstellung von Riboflavin mittels Mikroorgaismen mit veraenderter Isocitratlyase Aktivitaet
   (iii) ANZAHL DER SEQUENZEN: 2
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 2364 Basenpaare
      (B) ART: Nukleins"ure
      (C) STRANGFORM: Doppel
      (D) TOPOLOGIE: linear
   (ii) ART DES MOLEKÄLS: cDNS zu mRNS
   (iii) HYPOTHETISCH: NEIN
   (iii) ANTISENSE: NEIN
   (ix) MERKMALE:
      (A) NAME/SCHLÜSSEL: 5'UTR
      (B) LAGE: 1..550
   (ix) MERKMALE:
      (A) NAME/SCHLÜSSEL: CDS
      (B) LAGE: 551..2233
   (ix) MERKMALE:
      (A) NAME/SCHLÜSSEL: 3'UTR
      (B) LAGE: 2234..2364
   (xi) SEQUENZBESCHREIBUNG: SEQ ID NO: 1:
   CGAAAGCGCC AAATACCGGA AACGGCACAG GCGCAGCTCT AATAGCCGTT CCACGATAAC      60
   TTTGGAAGTT ATGGCACTAT GGCCGAGTGG TTAAGGCGAC AGACTTGAAA TCTGTTGGGC     120
   TCTGCCCGCG CTGGTTCAAA TCCTGCTGGT GTCGTTATTT TTGCCGTTTC TTTTTAGATG     180
   AAACTCAGGG GCCTTTAGTC CGCCCTTTTG CCCGCTGATT CATCGCCCGC CAGCAACACC     240
   GGTTGAGCCG ATCAGCGCAA GAACGCGCAA AGTCACGTAT GGCCCCTAAG AGTTGAGCTC     300
   TCCCCCTCGG CTCCTTCCGG GCGCGGAAAA GCCTGCGTCA CCCCATTAAG TCCGAAACCG     360
   CGTTCAAGTG TACTTGGTCC GGGCCAATGT GGTTGCCTCA TCCGAGTCAC CGATACGCAG     420
   GTGCGCCCGT CGAGTCACCA TTAGGAGTAG AGCATCTGAT TATATATAGG CCTAGTTACA     480
   GCGGTAACAT AGACTGATAG CTCCAGCTCC AGCACTAGCT TGTAGGACAT CTGCGCGACA     540
   CCCAGTGAAC ATG TCC CCT TCC GTC AGA GAC GCC CGC AAC GAC CTT GCC      589
              Met Ser Pro Ser Val Arg Asp Ala Arg Asn Asp Leu Ala
               1               5                  10
   AGC CTG CAA CAG CAG GCA GCC GCC GAA GCC GAG GAT ATT AGG AGA TGG      637
   Ser Leu Gln Gln Gln Ala Ala Ala Glu Ala Glu Asp Ile Arg Arg Trp
        15                  20                  25
   TGG AGC CAG CCA CGG TGG GCG GGC ACC AAG CGC GTG TAC ACG GCC GAG      685
   Trp Ser Gln Pro Arg Trp Ala Gly Thr Lys Arg Val Tyr Thr Ala Glu
    30                  35                  40                  45
   GAC ATC GTC AAG CGC CGC GGC ACG TTC CCT GTC GTC GAA TAC CCA TCT      733
   Asp Ile Val Lys Arg Arg Gly Thr Phe Pro Val Val Glu Tyr Pro Ser
                    50                  55                  60
   TCC GTA ATG GCG GAC AAG CTC GTG GAG ACA TTG GCG CGG CAC TCG CGC      781
   Ser Val Met Ala Asp Lys Leu Val Glu Thr Leu Ala Arg His Ser Arg
                65                  70                  75
   AAC GGC ACG GTT TCA CAG ACG TTC GGA GTG CTC GAC CCA GTG CAA ATG      829
   Asn Gly Thr Val Ser Gln Thr Phe Gly Val Leu Asp Pro Val Gln Met
            80                  85                  90
   ACG CAA ATG GTG AAG TAT CTG GAC ACG ATT TAC GTG TCT GGC TGG CAA      877
   Thr Gln Met Val Lys Tyr Leu Asp Thr Ile Tyr Val Ser Gly Trp Gln
        95                 100                 105
   TGC AGC GCC ACG GCT TCG ACC TCG AAC GAG CCT GGG CCC GAT CTC GCG      925
   Cys Ser Ala Thr Ala Ser Thr Ser Asn Glu Pro Gly Pro Asp Leu Ala
   110                 115                 120                 125
   GAC TAT CCG ATG GAC ACC GTG CCA AAC AAG GTC GAG CAC CTG TTC ATG      973
   Asp Tyr Pro Met Asp Thr Val Pro Asn Lys Val Glu His Leu Phe Met
                   130                 135                 140
   GCG CAG CTG TTC CAC GAC CGG AAA CAG CGC GAG GCC CGC CTG TCG TGC      1021
   Ala Gln Leu Phe His Asp Arg Lys Gln Arg Glu Ala Arg Leu Ser Cys
               145                 150                 155
   ACT ACC CAG CGC GAG CTC GAC CAA TTG GGG CCT GAG ATT GAC TAC TTG      1069
   Thr Thr Gln Arg Glu Leu Asp Gln Leu Gly Pro Glu Ile Asp Tyr Leu
           160                 165                 170
   AGG CCG ATT GTC GCT GAC GCA GAC ACC GGC CAC GGC GGG CTA ACA GCC      1117
   Arg Pro Ile Val Ala Asp Ala Asp Thr Gly His Gly Gly Leu Thr Ala
       175                 180                 185
   GTC TTT AAA CTC ACG AAG ATG TTC ATC GAG CGC GGT GCA GCC GGT ATC      1165
   Val Phe Lys Leu Thr Lys Met Phe Ile Glu Arg Gly Ala Ala Gly Ile
   190                 195                 200                 205
   CAC ATG GAG GAC CAG TCC TCC AGC AAC AAA AAG TGC GGG CAC ATG GCG      1213
   His Met Glu Asp Gln Ser Ser Ser Asn Lys Lys Cys Gly His Met Ala
                   210                 215                 220
   GGC CGC TGC GTG ATC CCT GTT CAG GAG CAC ATT AGT CGT TTA GTG ACT      1261
   Gly Arg Cys Val Ile Pro Val Gln Glu His Ile Ser Arg Leu Val Thr
               225                 230                 235
   GTG CGC ATG TGT GCG GAC GTG ATG CAC TCG AAC CTG GTG CTT GTC GCG      1309
   Val Arg Met Cys Ala Asp Val Met His Ser Asn Leu Val Leu Val Ala
           240                 245                 250
   AGA ACA GAC TCG GAG GCC GCC ACC TTA CTT AGC TCG AAC ATT GAC GCG      1357
   Arg Thr Asp Ser Glu Ala Ala Thr Leu Leu Ser Ser Asn Ile Asp Ala
       255                 260                 265
   CGC GAT CAT TAC TAC ATT GTC GGG GCC TCG AAC CCT GAG GTA ACT GTA      1405
   Arg Asp His Tyr Tyr Ile Val Gly Ala Ser Asn Pro Glu Val Thr Val
   270                 275                 280                 285
   CCG CTG ATC GAA GTT TTG GAC GCC GCG CAG CAG GCC GGC GCC TCA GGT      1453
   Pro Leu Ile Glu Val Leu Asp Ala Ala Gln Gln Ala Gly Ala Ser Gly
                   290                 295                 300
   GAC AGA TTG GCT CAG CTA GAG GAG GAC TGG TGC AAG AAG GCC AAG TTG      1501
   Asp Arg Leu Ala Gln Leu Glu Glu Asp Trp Cys Lys Lys Ala Lys Leu
               305                 310                 315
   AGG CTC TTC CAC GAG GCA TTT GCC GAC CAG GTG AAT GCC AGC CCT TCG      1549
   Arg Leu Phe His Glu Ala Phe Ala Asp Gln Val Asn Ala Ser Pro Ser
           320                 325                 330
   ATC AAA GAC AAG GCG GGC GTT ATT GCC AAA TTT AAC TCA CAG ATC GGG      1597
   Ile Lys Asp Lys Ala Gly Val Ile Ala Lys Phe Asn Ser Gln Ile Gly
       335                 340                 345
   CCA CAG ACA GGC GCG TCG ATC AGA GAG ATG CGC AAA CTG GGC CGC GAG      1645
   Pro Gln Thr Gly Ala Ser Ile Arg Glu Met Arg Lys Leu Gly Arg Glu
   350                 355                 360                 365
   CTG CTC GGG CAG GAC GTC TAC TTC GAC TGG GAC CTG CCT CGC GCT AGA      1693
   Leu Leu Gly Gln Asp Val Tyr Phe Asp Trp Asp Leu Pro Arg Ala Arg
                   370                 375                 380
   GAG GGC TTG TAC CGC TAC AAG GGC GGC ACC CAG TGC GCG ATC ATG CGC      1741
   Glu Gly Leu Tyr Arg Tyr Lys Gly Gly Thr Gln Cys Ala Ile Met Arg
               385                 390                 395
   GCA CGC GCG TTC GCG CCG TAC GCC GAC CTG GTC TGG TTC GAA TCC AAC      1789
   Ala Arg Ala Phe Ala Pro Tyr Ala Asp Leu Val Trp Phe Glu Ser Asn
           400                 405                 410
   TTC CCT GAC TTC CAG CAG GCT AAG GAG TTT GCG CAG GGC GTG CGC GAG      1837
   Phe Pro Asp Phe Gln Gln Ala Lys Glu Phe Ala Gln Gly Val Arg Glu
       415                 420                 425
   AAG TTC CCC AAC AAG TGG ATG GCC TAC AAC TTG TCG CCC AGC TTC AAC      1885
   Lys Phe Pro Asn Lys Trp Met Ala Tyr Asn Leu Ser Pro Ser Phe Asn
   430                 435                 440                 445
   TGG CCG AAG GCC ATG CCT CCC AAG GAG CAG GAG AAC TAC ATC CAA CGG      1933
   Trp Pro Lys Ala Met Pro Pro Lys Glu Gln Glu Asn Tyr Ile Gln Arg
                   450                 455                 460
   CTG GGC GAG ATC GGA TAT GTG TGG CAG TTC ATC ACG CTA GCC GGC CTG      1981
   Leu Gly Glu Ile Gly Tyr Val Trp Gln Phe Ile Thr Leu Ala Gly Leu
               465                 470                 475
   CAT ACC AAT GCC TTG GCC ATC GAC AAC TTC TCG CGC GAA TTC AGC AGG      2029
   His Thr Asn Ala Leu Ala Ile Asp Asn Phe Ser Arg Glu Phe Ser Arg
           480                 485                 490
   TTC GGA ATG CGT GCG TAT GCA CAA GGC ATC CAG CAG AGG GAG ATG GAC      2077
   Phe Gly Met Arg Ala Tyr Ala Gln Gly Ile Gln Gln Arg Glu Met Asp
       495                 500                 505
   GAG GGC GTC GAT GTC CTA AAA CAC CAG AAG TGG GCC GGC GCA GAG TAT      2125
   Glu Gly Val Asp Val Leu Lys His Gln Lys Trp Ala Gly Ala Glu Tyr
   510                 515                 520                 525
   GTT GAC AGC ATT CTC AAG CTT GCC CAG GGC GGT GTG TCT TCG ACA GCC      2173
   Val Asp Ser Ile Leu Lys Leu Ala Gln Gly Gly Val Ser Ser Thr Ala
                   530                 535                 540
   TCG ATG GGT AAG GGT GTA ACC GAA GAG CAG TTC GGC TCC TCA AAC GGT      2221
   Ser Met Gly Lys Gly Val Thr Glu Glu Gln Phe Gly Ser Ser Asn Gly
               545                 550                 555
   GCC AAA CTA TGATATCATC TCTGAGTCAT TTCTCTCGAC AAGATCCTCG              2270
   Ala Lys Leu
           560
   GCCAGACTTC TGGAATATAT ATAACATCGG GTACCCCGAC ATCCCTGCCT TCCGCAACGT    2330
   GCGAAGCAGC TGATACGTAT ACTTTAAACG CACA                                2364
(2) INFORMATION ZU SEQ ID NO: 2:
   (i) SEQUENZ CHARAKTERISTIKA:
      (A) LÄNGE: 560 Aminosäuren
      (B) ART: Aminosäure
      (D) TOPOLOGIE: linear
   (ii) 'ART DES MOLEKÜLS: Protein
   (xi) SEQUENZBESCHREIBUNG: SEQ ID NO: 2:
   Met Ser Pro Ser Val Arg Asp Ala Arg Asn Asp Leu Ala Ser Leu Gln
    1               5                  10                  15
   Gln Gln Ala Ala Ala Glu Ala Glu Asp Ile Arg Arg Trp Trp Ser Gln
               20                  25                  30
   Pro Arg Trp Ala Gly Thr Lys Arg Val Tyr Thr Ala Glu Asp Ile Val
           35                  40                  45
   Lys Arg Arg Gly Thr Phe Pro Val Val Glu Tyr Pro Ser Ser Val Met
       50                  55                  60
   Ala Asp Lys Leu Val Glu Thr Leu Ala Arg His Ser Arg Asn Gly Thr
   65                  70                  75                  80
   Val Ser Gln Thr Phe Gly Val Leu Asp Pro Val Gln Met Thr Gln Met
                   85                  90                  95
   Val Lys Tyr Leu Asp Thr Ile Tyr Val Ser Gly Trp Gln Cys Ser Ala
               100                 105                 110
   Thr Ala Ser Thr Ser Asn Glu Pro Gly Pro Asp Leu Ala Asp Tyr Pro
           115                 120                 125
   Met Asp Thr Val Pro Asn Lys Val Glu His Leu Phe Met Ala Gln Leu
       130                 135                 140
   Phe His Asp Arg Lys Gln Arg Glu Ala Arg Leu Ser Cys Thr Thr Gln
   145                 150                 155                 160
   Arg Glu Leu Asp Gln Leu Gly Pro Glu Ile Asp Tyr Leu Arg Pro Ile
                   165                 170                 175
   Val Ala Asp Ala Asp Thr Gly His Gly Gly Leu Thr Ala Val Phe Lys
               180                 185                 190
   Leu Thr Lys Met Phe Ile Glu Arg Gly Ala Ala Gly Ile His Met Glu
           195                 200                 205
   Asp Gln Ser Ser Ser Asn Lys Lys Cys Gly His Met Ala Gly Arg Cys
       210                 215                 220
   Val Ile Pro Val Gln Glu His Ile Ser Arg Leu Val Thr Val Arg Met
   225                 230                 235                 240
   Cys Ala Asp Val Met His Ser Asn Leu Val Leu Val Ala Arg Thr Asp
                   245                 250                 255
   Ser Glu Ala Ala Thr Leu Leu Ser Ser Asn Ile Asp Ala Arg Asp His
               260                 265                 270
   Tyr Tyr Ile Val Gly Ala Ser Asn Pro Glu Val Thr Val Pro Leu Ile
           275                 280                 285
   Glu Val Leu Asp Ala Ala Gln Gln Ala Gly Ala Ser Gly Asp Arg Leu
       290                 295                 300
   Ala Gln Leu Glu Glu Asp Trp Cys Lys Lys Ala Lys Leu Arg Leu Phe
   305                 310                 315                 320
   His Glu Ala Phe Ala Asp Gln Val Asn Ala Ser Pro Ser Ile Lys Asp
                   325                 330                 335
   Lys Ala Gly Val Ile Ala Lys Phe Asn Ser Gln Ile Gly Pro Gln Thr
               340                 345                 350
   Gly Ala Ser Ile Arg Glu Met Arg Lys Leu Gly Arg Glu Leu Leu Gly
           355                 360                 365
   Gln Asp Val Tyr Phe Asp Trp Asp Leu Pro Arg Ala Arg Glu Gly Leu
       370                 375                 380
   Tyr Arg Tyr Lys Gly Gly Thr Gln Cys Ala Ile Met Arg Ala Arg Ala
   385                 390                 395                 400
   Phe Ala Pro Tyr Ala Asp Leu Val Trp Phe Glu Ser Asn Phe Pro Asp
                   405                 410                 415
   Phe Gln Gln Ala Lys Glu Phe Ala Gln Gly Val Arg Glu Lys Phe Pro
               420                 425                 430
   Asn Lys Trp Met Ala Tyr Asn Leu Ser Pro Ser Phe Asn Trp Pro Lys
           435                 440                 445
   Ala Met Pro Pro Lys Glu Gln Glu Asn Tyr Ile Gln Arg Leu Gly Glu
       450                 455                 460
   Ile Gly Tyr Val Trp Gln Phe Ile Thr Leu Ala Gly Leu His Thr Asn
   465                 470                 475                 480
   Ala Leu Ala Ile Asp Asn Phe Ser Arg Glu Phe Ser Arg Phe Gly Met
                   485                 490                 495
   Arg Ala Tyr Ala Gln Gly Ile Gln Gln Arg Glu Met Asp Glu Gly Val
               500                 505                 510
   Asp Val Leu Lys His Gln Lys Trp Ala Gly Ala Glu Tyr Val Asp Ser
           515                 520                 525
   Ile Leu Lys Leu Ala Gln Gly Gly Val Ser Ser Thr Ala Ser Met Gly
       530                 535                 540
   Lys Gly Val Thr Glu Glu Gln Phe Gly Ser Ser Asn Gly Ala Lys Leu
   545                 550                 555                 560